(19) Europäisches Patentamt
European Patent Office
Office européen des brevets

(11) EP 0 572 776 B1

(12) **EUROPÄISCHE PATENTSCHRIFT**

(45) Veröffentlichungstag und Bekanntmachung des Hinweises auf die Patenterteilung:
**21.08.1996 Patentblatt 1996/34**

(51) Int. Cl.[6]: **A61K 7/50**, A61K 7/08

(21) Anmeldenummer: **93105100.7**

(22) Anmeldetag: **27.03.1993**

(54) **Elektrolytverdickbare Tensidkombinationen**

Electrolyte thickenable detergent composition

Composition d'agent tensio-actifs épaississant par électrolyte

(84) Benannte Vertragsstaaten:
**AT BE CH DE DK ES FR GB GR IT LI LU NL PT SE**

(30) Priorität: **29.05.1992 DE 4217673**

(43) Veröffentlichungstag der Anmeldung:
**08.12.1993 Patentblatt 1993/49**

(73) Patentinhaber: **HÜLS AKTIENGESELLSCHAFT**
**D-45764 Marl (DE)**

(72) Erfinder: **Balzer, Dieter, Dr.**
**W-4358 Haltern (DE)**

(56) Entgegenhaltungen:
**EP-A- 0 384 983**
**EP-A- 0 507 047**
**EP-A- 0 511 466**
**WO-A-91/15192**
**WO-A-93/04662**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 572 776 B1

Printed by Rank Xerox (UK) Business Services
2.13.2/3.4

## Beschreibung

Die Erfindung betrifft mittels Elektrolyt verdickbare Tensidkombinationen als Basis für Shampoonierungsmittel, Bade- und Duschgele sowie andere kosmetische wäßrige Reinigungsflüssigkeiten, für die aus Applikationsgründen eine erhöhte Viskosität notwendig bzw. wünschenswert ist.

In der Vergangenheit basierten solche Systeme vorwiegend auf Alkylsulfaten oder Alkylethersulfaten mit gewöhnlich niedrigem Ethoxylierungsgrad. Die Viskosität hierbei läßt sich relativ einfach durch Zugabe von Kochsalz, Ammoniumchlorid, eventuell kombiniert mit Fettsäurediethaflolamid, und/oder anderen Additiven einstellen. Solche Formulierungen zeigen zwar befriedigenden Schaum und sind preiswert, haben allerdings den großen Nachteil starker Hautreizung sowie Augenschleimhautreizung, was in Anbetracht der immer häufiger werdenden, zum Teil täglichen Anwendungen von erheblichem Gewicht ist. Aufgrund des Risikopotentials von Spurenverunreinigungen an N-Nitrosodiethanolamin ist es darüber hinaus sinnvoll, möglichst N-freie Formulierungen zur Verfügung zu haben [vgl. Hamke Meijer, Seifen-Öle-Fette-Wachse 114, 159 (1988)].

Die Suche nach milderen Tensidpräparationen, die nicht die o. a. Nachteile beinhalten, sich aber gleichzeitig einfach verdicken lassen, ist somit angezeigt. Es taucht hierbei das Problem auf, daß haut- bzw. schleimhautfreundliche, preiswerte Tenside meist keine genügende Elektrolytverdickbarkeit [H. Meijer, Seifen-Öle-Fette-Wachse 113, 135 (1987) und H. Tesmann, Parfümerie und Kosmetik 68, 630 (1987)] aufweisen. Ausreichend hohe Viskosität bzw. Verdickbarkeit versucht man daher entweder durch Erhöhung der Tensidkonzentration oder mittels einer nur sehr begrenzten Teilsubstitution des Ethersulfates durch ein milderes und toxikologisch unbedenklicheres Tensid zu erzielen (H. Meijer, loc. cit und US-PS 3 038 862), was den eben aufgeführten Nachteilen aber nicht gerecht wird. Eine Verdickung mittels wasserlöslicher Polymerer gilt hierbei wegen der Beeinflussung der Schaumqualität sowie des Hautgefühls als ungeeignete Alternative.

Als milde, haut- und schleimhautverträgliche Tenside gelten seit langem die Salze der ethoxylierten Sulfobernsteinsäurehalbester [oder auch Sulfosuccinate (H. Hoffmann, Fette-Seifen-Anstrichmittel 65, 748 (1963))]. Wie in den Beispielen gezeigt wird, besitzen sie eine hohe Elektrolytverrträglichkeit, gelten aber als nicht verdickbar. Dies gilt allerdings nur für solche Elektrolytkonzentrationen, wie sie für Personal Care Formulieerungen üblich sind (bis zu etwa 5 % NaCl). In Gegenwart wesentlich höherer Elektrolytkonzentrationen, die aber ihrerseits bereits erheblich hautirritierend sind, werden hingegen hohe Viskositäten erreicht.

Die Kombination von Alkylpolyglykosiden mit Sulfosuccinaten allein ergibt zwar gut verdickbare Kompositionen, das Hydrolysepotential der Sulfosuccinate erfordert aber die Einstellung eines pH von ungefähr 6 bis 8, so daß alleinige Kombinationen von Sulfosuccinaten mit Alkylpolyglykosiden im schwach sauren Bereich nicht stabil genug sind (WO 90/14411).

Ein praktisch identisches Bild sowohl hinsichtlich Haut- und Schleimhautverträglichkeit als auch hinsichtlich Elektrolytverträglichkeit und Verdickung wird bei carboxymethylierten Fettalkoholoxethylaten beobachtet [N.A.I. van Paasen, Seifen-Öle-Fette-Wachse 109, 353 (1983)]. Auch sie sind bei üblichen Neutralelektrolytkonzentrationen nicht oder nur unzureichend verdickbar (EP-A 0 176 151), erreichen bei NaCl-Konzentrationen von 15 % jedoch sehr hohe Viskositäten.

Eine gewisse Lösung des Problems der elektrolytischen Verdickung hautfreundlicher Tenside gelingt jedoch durch eine strukturelle Versänderung der zuletzt genannten Tensidklasse. So wurde gezeigt, daß carboxymethylierte Fettalkoholoxethylate mit ungesättigten Alkylgruppen mittels üblicher Elektrolytkonzentrationen gut verdickbar sind (US-PS 5 098 596). Von großem Nachteil hierbei sind jedoch wesentlich höhere Rohstoffkosten sowie eine weitaus schwierigere Synthese.

Alkylpolyglycoside oder Alkyloligoglycoside in Kombination mit anionischen Tensiden wurden ebenfalls als Personal Care Formulierungen vorgeschlagen.

So beschreibt bereits die DRP 593 422 die Verwendung von Cetylmaltosid mit gewöhnlicher Seife, US-PS 3 721 633 betrifft die Verwendung von Gemischen aus Alkylpolyglycosiden mit anionischen synthetischen Tensiden, wie z. B. Dodecylbenzolsulfonat, und das Technical Bulletin Triton CG 110 der Rohm und Haas von 1975 erwähnt eine Abmischung des Alkylpolyglycosids mit u. a. Laurylethersulfat.

WO 86/02943 beschreibt die Kombination von Alkylmonoglycosiden bzw. Alkyloligoglycosiden mit unterschiedlichen Aniontensiden wie Alkylsulfat, Alkylethersulfat, Olefinsulfonat, Paraffinsulfonat oder Alkylbenzolsulfonat mit dem Ziel einer Viskositätserhöhung. Im Unterschied zur vorliegenden Erfindung sind die beanspruchten Aniontenside wenig haut- und schleimhautverträglich, was von erheblichem Gewicht ist.

Die Kombination von Alkylpolyglycosiden mit beliebigen anionischen Tensiden sowie deren Gemischen in nahezu beliebigen Verhältnissen wird von EP-A 0 070 074 beschrieben, wobei eine Elektrolytverdickbarkeit aber keine Erwähnung findet.

Die Verwendung eines speziellen Verdickersystems, das aus Alkylpolyglucosid, insbesondere Derivaten alkoxylierter mehrwertiger Alkohole mit 2 bis 8 C-Atomen wie Ethylenglycol, Propandiol, Glycerin, Butandiol, Erythrit, Pentaerythrit, Arabit, Sorbit usw. und gegebenenfalls Fremdelektrolyt besteht, beschreibt EP-A-0 511 466. Dieses verdickte System benötigt also eine zusätzliche, tensidfremde Komponente.

Und schließlich beschreibt EP-A 0 358 216 die Verwendung von Kombinationen aus Alkylpolyglycosiden mit Sulfosuccinaten in beliebigen Verhältnissen für gering reizende Detergenzien, wobei eine Viskositätsanpassung mittels Elektrolyt aber keine Rolle spielt.

Eine andere Lösung des Problems gelingt nach EP-A 0 384 983 durch Tensidkombinationen bestehend aus carboxymethylierten Fettalkoholoxethylaten und Alkylpolyglycosiden, ebenfalls einer sehr hautfreundlichen, jedoch nichtionischen Tensidklasse, die selbst nicht elektrolytverdickbar ist. Nachteilig ist hierbei aber, daß die carboxymethylierten Oxethylate wenigstens zu einem Teil, etwa 50 % ungesättigt sein müssen, was zu den gleichen negativen Konsequenzen führt, wie oben dargestellt.

Es bestand daher weiterhin die Aufgabe haut- und schleimhautfreundliche Tensidkombinationen für kosmetische Anwendungen aufzufinden, die synthetisch leicht und preiswert zugänglich sind und die sich mit möglichst niedrigen Elektrolytkonzentrationen befriedigend verdicken lassen.

Diese Aufgabe wird gelöst durch eine Tensidkombination enthaltend

a) 7 bis 25 Gew-.% Alkylpolyglycosid
b) 3 bis 25 Gew.-% carboxymethyliertes Alkanoloxethylat
c) 0 bis 25 Gew-.% Monoalkylsulfosuccinat und
d) 0 bis 3 % tensidische Additive,

in Wasser, wobei bestimmte enge Mengenverhältnisse von a : b : c eingesetzt werden.

Gegenstand der Erfindung sind elektrolytverdickbare Tensidkombinationen für Badegele, Shampoos und kosmetische Reinigungsmittel enthaltend

a) 7 bis 25 Gew-.% Alkylpolyglycosid
b) 3 bis 25 Gew.-% carboxymethyliertes Alkanoloxethylat
c) 0 bis 25 Gew-.% Monoalkylsulfosuccinat
d) 0 bis 3 % tensidische Additive,

dadurch gekennzeichnet, daß sich die Mengen a : b bzw. a : (b + c) wie 4 : 6 bis 7 : 3 verhalten, das Verhältnis (a + b) : d bzw. (a + b + c) : d 5 nicht unterschreitet und b : c sich verhalten wie 1 : 8 bis 10 : 0.

Es ist völlig unerwartet und überraschend, daß Kombinationen aus Alkylpolyglycosid und carboxymethyliertem Alkanoloxethylat allein oder carboxymethyliertem Alkanoloxethylat und Monoalkylsulfosuccinat bei Vorliegen ganz bestimmter Mengenverhältnisse eine ideale elektrolytische Verdickung zeigen, wie in den erfindungsgemäßen Beispielen gezeigt wird.

Alkylpolyglycoside:

Erfindungsgemäß eingesetzte Glycoside sind Alkylpolyglycoside, das heißt Verbindungen der Formel (I)

$$R\text{-}O\text{-}Z_n \qquad (I),$$

in der R für einen linearen oder verzweigten gesättigten oder ungesättigten aliphatischen Alkylrest mit 8 bis 18 Kohlenstoffatomen oder Gemische davon und $Z_n$ für einen Oligoglycosylrest mit n = 1 bis 2 steht. Bevorzugt werden Alkylpolyglucoside mit einem Alkylrest von 10 bis 16 Kohlenstoffatomen und einem Glucosidierungsgrad von 1,1 bis 1,6.

Die erfindungsgemäß eingesetzten Alkylpolyglycoside können nach bekannten Verfahren ganz oder teilweise auf Basis nachwachsender Rohstoffe hergestellt werden. Beispielsweise wird Dextrose in Gegenwart eines sauren Katalysators mit n-Butanol zu Butyloligoglycosidgemischen umgesetzt, welche mit langkettigen Alkoholen in Gegenwart eines ebenfalls sauren Katalysators zu den gewünschten Alkyloligoglycosidgemischen umglykosidiert werden. Die Formel der Produkte ist in bestimmten Grenzen variierbar. Der Alkylrest R wird durch die Auswahl des langkettigen Alkohols festgelegt. Günstig aus wirtschaftlichen Gründen sind die großtechnisch zugänglichen Tensidalkohole mit 8 bis 18 C-Atomen, z. B. native Alkohole, Ziegleralkohole und Oxoalkohole.

Der Oligoglycosylrest $Z_n$ wird einerseits durch die Auswahl des Kohlenhydrats und andererseits durch die Einstellung des mittleren Polymerisationsgrades n z. B. nach DE-OS 19 43 689 festgelegt. Im Prinzip können bekanntlich Polysaccharide, Oligosaccharide und Monosaccharide, z. B. Stärke, Maltodextrine, Dextrose, Galaktose, Mannose, Xylose usw. zu Alkyloligoglycosiden umgesetzt werden. Besonders bevorzugt sind die großtechnisch verfügbaren Kohlenhydrate Stärke, Maltodextrine und Dextrose. Da die wirtschaftlich interessanten Alkylpolyglycosidsynthesen nicht regio-und stereoselektiv verlaufen, sind die Alkylpolyglycoside stets Gemische von Oligomeren, die ihrerseits Gemische verschiedener isomerer Formen darstellen. Sie liegen nebeneinander mit α- und β-glycosidischen Bindungen in Pyranose- und Furanoseform vor. Auch die Verknüpfungsstellen zwischen zwei Saccharidresten sind unterschiedlich.

Erfindungsgemäß eingesetzte Alkyloligoglycoside lassen sich auch durch Abmischen von Alkylpolyglycosiden mit Alkylmonoglycosiden herstellen. Letztere kann man z. B. nach EP-A 0 092 355 mittels polarer Lösemittel wie Aceton aus Alkylpolyglycosiden gewinnen bzw. anreichern.

Der Glycosidierungsgrad wird zweckmäßigerweise mittels $^1$H-NMR bestimmt.

Im Vergleich zu allen anderen in kosmetischen Reinigungsmitteln eingesetzten Tensiden gelten die Alkylpolyglycoside als überaus umweltverträglich. So liegt der mittels Kläranlagen-Simulationsmodell/DOC-Analyse bestimmte biologische Abbaugrad für die erfindungsgemäßen Alkylpolyglycoside bei 99 ± 2 %. Diese Zahl ist vor dem Hintergrund zu sehen, daß bei diesem Testverfahren (Totalabbau) bereits ein Abbaugrad > 70 % die Substanz als gut abbaubar charakterisiert.

Auch die akute orale Toxizität LD 50 (Ratte) sowie die aquatische Toxizität LC 50 (Goldorfe) und EC 50 (Daphnien) mit Werten von > 10 000 mg/kg und 12 bzw. 30 mg/l liegen um den Faktor 3 bis 5 günstiger als die entsprechenden Werte der heute wichtigsten Tenside. Ähnliches gilt für die bei kosmetischen Formulierungen besonders wichtige Haut- und Schleimhautverträglichkeit.

Carboxymethylierte Alkanoloxethylate:

Erfindungsgemäß eingesetzte carboxymethylierte Oxethylate entsprechen der Formel (II)

$$R'\text{-}(OC_2H_4)_mO\text{-}CH_2\text{-}COOM \qquad (II),$$

in der R' einen linearen oder verzweigten, gesättigten aliphatischen Rest mit 10 bis 18 Kohlenstoffatomen, m 1 bis 11 und M Alkali, Erdalkali, Wasserstoff, Ammonium oder Alkylammonium bedeuten. Bevorzugt werden carboxymethylierte Fettalkoholoxethylate mit 12 bis 16 Kohlenstoffatomen in dem linearen aliphatischen Rest, und mittleren Ethoxylierungsgraden von 2 bis 8, wobei auch Gemische verwendet werden können.

Die carboxymethylierten Oxethylate können z. B. nach der DE-PS 24 18 444 durch Umsetzung von Oxethylaten der Formel $R'\text{-}(OC_2H_4)_mH$ mit einem Salz der Chloressigsäure in Gegenwart von Alkalihydroxid oder anderen Basen hergestellt werden. Die Umsetzung muß hierbei nicht quantitativ sein, so daß das carboxymethylierte Oxethylat ein Gemisch von Ausgangsoxethylat und Umsetzungsprodukt ist. Erfindungsgemäß ist ein Umsetzungsgrad zwischen 60 und 100 %, bevorzugt werden 70 bis 100 %.

Monoalkylsulfosuccinate:

Erfindungsgemäß eingesetzte Monoalkylsulfosuccinate entsprechen der Formel (III)

$$R''\text{-}O\text{-}CO\text{-}CH_{(2)}\text{-}CH_{(2)}\text{-}COOMe \qquad (III),$$
$$\quad | $$
$$SO_3Me$$

in der
R'' ein gesättigter oder ungesättigter, unverzweigter oder verzweigter Alkylrest mit 8 bis 18 Kohlenstoffatomen oder ein Alkyloxethylat der Formel (IV)

$$R'''(OCH_2CH_2)_p\text{-} \qquad (IV)$$

ist,
in der R''' wiederum ein gesättigter, verzweigter oder unverzweigter Rest mit 10 bis 18 Kohlenstoffatomen und p 1 bis 8 ist, und Me Alkali, Erdalkali, Wasserstoff, Ammonium oder Alkylammonium bedeutet.

Bevorzugt werden Natrium-Sulfosuccinate der Oxethylate von Fettalkoholen mit 12 bis 18 C-Atomen in der linearen, gesättigten Alkylkette und Ethoxylierungsgraden p zwischen 2 und 6.

Die erfindungsgemäßen Mengenverhältnisse der Komponenten sind kritisch. Ein Viskositätsoptimum bei schönem Schäumverhalten, wie es für viele Personal Care Anwendungen gefordert wird, wird dann beobachtet, wenn das Verhältnis a : b oder a : (b + c) zwischen 4 : 6 und 7 : 3 liegt. Bevorzugt ist ein Verhältnis zwischen 5 : 5 und 6,5 : 3,5. Das Verhältnis b : c ist relativ unkritisch, es sollte zwischen 1 : 8 und 10 : 0 liegen.

Tensidische Additive:

Als tensidische Additive kommen kleine Mengen schaumstärkende, möglichst milde, verdickende und konditionierende Verbindungen in Frage. Als Beispiele genannt seien Fettalkoholsulfate, Fettalkoholethersulfate, Alkansulfonate, Olefinsulfonate, Sarcosinate, Tauride, Alkylbetaine, Alkylamidopropylbetaine, Eiweißhydrolysate, Fettalkoholoxethylate, Fettalkoholoxethylatsorbitanester, ethoxylierte und nicht ethoxylierte Fettsäureamide, Trialkylaminoxide, Silikontenside, quaternäre Tenside, Di- und Oligofettsäureester von hochethoxylierten mehrwertigen Alkoholen, sowie deren Gemische.

Der Gehalt dieser die eigentliche Tensidkombination korrigierenden Additive soll maximal 3 % bezogen auf die Endformulierung betragen, wobei das Verhältnis ( a + b + c) : d 5 nicht unterschreiten soll.

Die Tensidkombination zeichnet sich durch eine hervorragende Verdickbarkeit bei bereits geringen Konzentrationen von Elektrolyt aus. Als solche eignen sich z. B. Natriumchlorid, Ammoniumchlorid, Natriumsulfat, Magnesiumsulfat etc. in Konzentrationen zwischen 0,5 und 5 %, bevorzugt wird NaCl mit Konzentrationen zwischen 1 und 3 %.

Weitere Bestandteile sind solche, wie sie für Personal Care Formulierungen üblich sind, wie Duftstoffe auf pflanzlicher oder synthetischer Basis, Farbstoffe, Trübungs- und Perlglanzmittel, Rückfetter auf Paraffin-, Fett- oder Glyceridbasis, Silikonöle, Feuchthaltemittel, verdickende und konditionierende Polymere wie z. B. Xanthane, Cellulosederivate, etc. , kationische Proteinderivate, etc., hautkosmetische Wirkstoffe, Puffersubstanzen, wobei insbesondere bei sulfosuccinathaltigen Formulierungen pH-Bereiche zwischen 4 und 8 einzustellen sind, Komplexbildner, Konservierungsmittel, etc.

Mit den folgenden Beispielen soll die wirksame Verdickbarkeit der erfindungsgemäßen Kombination aufgezeigt werden. Sie wird nachgewiesen durch Viskositätsmessungen in einem Rotationsviskosimeter (Haake RV 20) bei 25 °C unter definierten Scherraten. Bei stark strukturviskosen Zubereitungen werden die mittleren Viskositäten bei Scherraten zwischen 5 und 10 $s^{-1}$ mitgeteilt, bei Bedingungen also, die in etwa dem Bewegungsvorgang beim Ausfluß einer Flüssigkeit aus einer Shampoo-Flasche mit mittlerer Öffnung entsprechen.

Gemessen wurde in manchen Fällen auch der Reibschaum nach Wilmsmann mit und ohne Sebum, der Klarpunkt sowie die Zeinzahl [Methoden beschrieben in D. Balzer, Tenside Surf. Det. 28, 419 (1991)].

Beispiel 1

In einem Glasgefäß werden 23 g $C_{12}C_{16}$-Alkylpolyglucosid mit einem Glucosidierungsgrad von 1,3 (52%ig in Wasser) sowie 8 g carboxymethyliertes $C_{12}C_{14}$-Fettalkoholoxethylat mit 4 mol Ethylenoxid/mol-Natriumsalz, Umsetzungsgrad 97 %, sowie 1,5 % NaCl mit 67,5 g Wasser verrührt. Die waschaktive Substanz beträgt 20 %. Das Gemisch wird leicht erwärmt, es ergibt sich eine auch nach Wochen klare Lösung. Die Viskositätsmessung ergibt einen Wert von ca. 5 000 mPa • s. Im Reibschaumtest ergibt sich ein feinporiger, stabiler, voluminöser Schaum, die Zeinzahl - ein Maß für die Hautirritation - liegt im Bereich der Wirkung von Wasser.

Die Werte sind ebenfalls in Abb. 1 dargestellt. In Abhängigkeit von der Zusammensetzung wird dort gezeigt, daß ein bei kosmetischen Formulierungen verwendbarer Viskositätspeak nur in einem schmalen Bereich (APG/carboxymethyliertes Oxethylat = 50/50 bis 70/30) existiert.

Beispiel 2

Unter identischen Bedingungen und mit gleichen Substanzen und Konzentrationen wie in Beispiel 1 werden der Tensidkombination (APG/carboxymethyliertes Oxethylat = 6 : 4) unterschiedliche NaCl-Mengen zugesetzt. Die Ergebnisse der Viskositätsmessungen werden in Abb. 2 Daten gegenübergestellt, die mit reinem carboxymethylierten Oxethylat erzielt werden. Der höchst unterschiedliche Kurvenverlauf (ca. 5 000 gegen 10 mPa • s) demonstriert den ungewöhnlich starken synergistischen Effekt bei Einhaltung der erfindungsgemäßen Mengenverhältnisse.

Beispiel 3

In einem Glasgefäß werden 26 g $C_{12}C_{14}$ $APG_{1,5}$ (46 % in Wasser) sowie 8 g carboxymethyliertes $C_{12}C_{14}$-Oxethylat mit 4 mol EO/mol Na-Salz, Umsetzungsgrad 97 % sowie 1 g Ammoniumchlorid in 65 g Wasser verrührt. Die Viskosität der klaren Lösung ergibt bei 25 °C einen Wert von 5 300 mPa • s. Alternativ ergibt eine Lösung von 20 g des o. a. carboxymethylierten Oxethylats und 1 g $NH_4Cl$ in 79 g Wasser eine Viskosität von 12 mPa • s. Die unterschiedlichen Ergebnisse zeigen den starken synergistischen Effekt der beiden Tenside, die als Einzelstoffe mit Elektrolyten bei realistischen, nicht irritierenden Konzentrationen bekanntlich nicht verdickbar sind (EP-A 0 384 983).

Beispiel 4

In einem Glasgefäß werden 20,8 g $C_{12}C_{14}$-APG mit einem Glucosidierungsgrad von 1,3 (48 % in Wasser) sowie je 5 g carboxymethylierte $C_{12}C_{14}$-Oxethylate mit 2 bzw. 4 mol EO/mol, Na-Salze (Umsetzungsgrade 95 bis 97 %) sowie 2 g NaCl in 65 g Wasser gelöst. Die Viskositätsmessung ergibt für die Lösung mit einem Aktivgehalt von 20 % und 25 °C, einen Wert von 6 500 mPa • s. Dieser Wert entspricht dem Viskositätsmaximum in Abb. 3. Die dort gezeigte Abhängigkeit von der Zusammensetzung dokumentiert den schmalen Bereich hoher Viskositäten, die für kosmetische Anwendungen interessant sind.

Beispiel 5

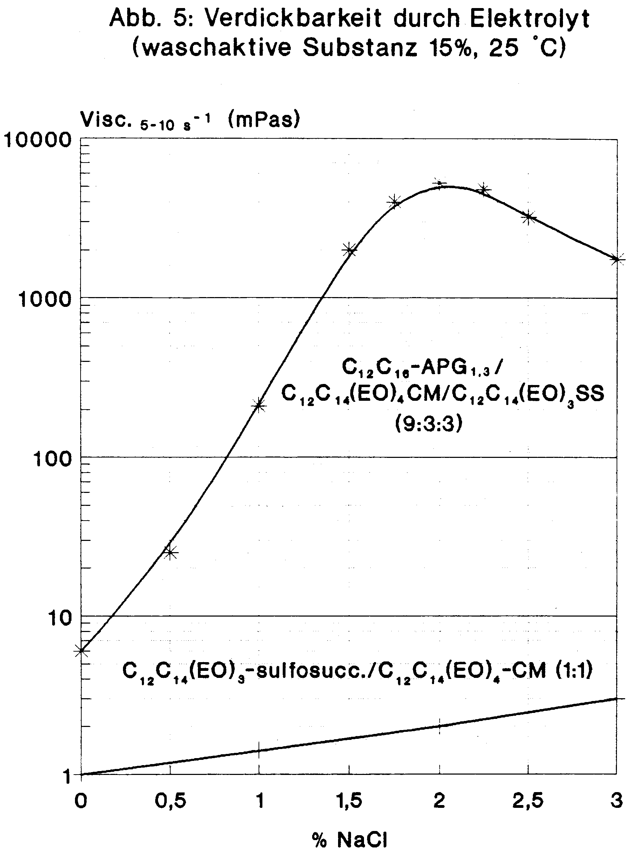

In einem Glasgefäß werden 18,8 g $C_{12}C_{14}$ $APG_{1,3}$ (48%ig in Wasser), 3 g carboxymethyliertes $C_{12}C_{14}$-Oxethylat mit 4 mol EO/mol Na-Salz, 8,8 g $C_{12}C_{14}$-Oxethylatsulfosuccinat Na-Salz mit 3 mol EO/mol (34%ig in Wasser), 5,2 g NaCl und 67,6 g Wasser zu einer klaren Lösung verrührt. Die Viskositätsmessung ergibt einen Wert von 4 000 mPa • s (vgl. Viskositätsmaximum Abb. 4). Die dort gezeigte Abhängigkeit von der Zusammensetzung zeigt auch hier deutlich den schmalen Mengenverhältnisbereich hoher Viskositäten, der interessant für kosmetische Formulierungen ist. Abb. 5 zeigt den dramatischen Vergleich der elektrolytischen Verdickung ohne und mit Alkylpolyglucosid (Verhältnis $C_{12}C_{14}$ $APG_{1,3}$/Sulfosuccinat bzw. carboxymethyliertes Oxethylat 6 : 4).

Alle Mengenangaben in den Abbildungen beziehen sich auf Gewichtsprozent, soweit nicht anders angegeben. Die Viskosität (25 °C) wird in mPa • s und der Reibschaum in ml bei gleichem Ordinatenmaßstab angegeben.

**Patentansprüche**

1. Elektrolytverdickbare Tensidkombinationen für Badegele, Shampoos und kosmetische Reinigungsmittel enthaltend

   a) 7 bis 25 Gew-.% Alkylpolyglycosid,
   b) 3 bis 25 Gew.-% carboxymethyliertes Alkanoloxethylat,
   c) 0 bis 25 Gew-.% Monoalkylsulfosuccinat,
   d) 0 bis 3 % tensidische Additive,

   dadurch gekennzeichnet,
   daß sich die Mengen a : b bzw. a: (b + c) wie 4 : 6 bis 7 : 3 verhalten, das Verhältnis (a + b) : d bzw. (a + b + c) : d 5 nicht unterschreitet und b : c sich verhält wie 1 : 8 bis 10 : 0 und

   a) als Alkylpolyglycoside Verbindungen der Formel (I)

   $$R\text{-}O\text{-}Z_n \quad (I),$$

   eingesetzt werden, wobei R einen gesättigten oder ungesättigten, verzweigten oder unverzweigten Alkylrest mit 8 bis 18 Kohlenstoffatomen und $Z_n$ ein Oligoglycosylradikal, bestehend aus 1 bis 2 Hexose- oder Pentoseeinheiten bzw. Mischungen davon, bedeutet,
   b) als carboxymethylierte Fettalkoholoxethylate Verbindungen der Formel (II)

   $$R'\text{-}(OC_2H_4)_mO\text{-}CH_2\text{-}COOM \quad (II)$$

   eingesetzt werden, in der R' einen linearen oder verzweigten, gesättigten aliphatischen Rest mit 10 bis 18 Kohlenstoffstomen, m 1 bis 11 und M ein Alkali-, Erdalkalimetall-, Wasserstoff-, Ammonium- oder Alkylammoniumion bedeutet, und wobei der Carboxymethylierungsgrad zwischen 60 und 100 Gew.-% liegt,
   c) als Monoalkylsulfosuccinate Verbindungen der Formel (III)

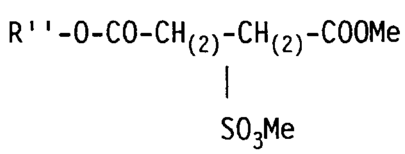

(III),

eingesetzt werden, in der R'' einen gesättigten oder ungesättigten, verzweigten oder unverzweigten Alkylrest mit 8 bis 18 Kohlenstoffatomen oder einen Alkylethoxyrest der Formel IV

$$R'''(OCH_2CH_2)_p- \qquad (IV),$$

in der R''' für einen gesättigten, verzweigten oder unverzweigten Alkylrest mit 10 bis 18 Kohlenstoffatomen, p für 1 bis 8 und Me für ein Alkali-, Erdalkali-, Wasserstoff-, Ammonium- oder Alkylammoniumion steht, bedeutet und d) als tensidische Additive organische Sulfate oder Ethersulfate, organische Sulfonate, Sarkosinate, Tauride, Betaine, Eiweißhydrolysate, quaternäre Tenside oder Di- und Oligofettsäureester von ethoxylierten mehrwertigen Alkoholen und all deren Mischungen zugesetzt werden.

**2.** Tensidkombination nach Anspruch 1,
dadurch gekennzeichnet,
daß als Alkylpolyglycosid ein Alkylglucosid mit einem gesättigten, unverzweigten Alkylrest mit 10 bis 16 Kohlenstoffatomen und einem Glucosidierungsgrad zwischen 1,1 und 1,6 verwendet wird.

**3.** Tensidkombination nach Anspruch 1,
dadurch gekennzeichnet,
daß R' einen linearen, gesättigten aliphatischen Rest mit 12 bis 16 Kohlenstoffatomen, m 2 bis 8 und M ein Alkali- oder Ammoniumion bedeutet, wobei der Carboxymethylierungsgrad 70 bis 100 % beträgt.

**4.** Tensidkombination nach Anspruch 1,
dadurch gekennzeichnet,
daß R'' ein Alkyloxethylat mit 12 bis 18 C-Atomen in der linearen, gesättigten Alkylkette ist, der Ethoxylierungsgrad 2 bis 6 beträgt und Me ein Alkali- oder Ammoniumion bedeutet.

**5.** Tensidkombination nach einem der Ansprüche 1 bis 4,
dadurch gekennzeichnet,
daß als elektrolytische Verdickungsmittel Natriumchlorid, Ammoniumchlorid, Natriumsulfat oder Magnesiumsulfat in Gehalten von 0,5 bis 5 % eingesetzt werden.

## Claims

**1.** An electrolyte-thickenable surfactant combination for bath gels, shampoos and cosmetic cleansers comprising

a) from 7 to 25% by weight of alkyl polyglycoside
b) from 3 to 25% by weight of carboxymethylated alkanol oxyethylate
c) from 0 to 25% by weight of monoalkyl sulphosuccinate
d) from 0 to 3% of surfactant additives,

characterized in that the amounts a:b and a:(b+c) are from 4:6 to 7:3, the ratio (a+b):d or (a+b+c):d is not less than 5 and b:c is from 1:8 to 10:0 and

a) as alkyl polyglycosides, compounds of the formula (I)

$$R\text{-}O\text{-}Z_n \qquad (I)$$

are employed, where R is a saturated or unsaturated, branched or unbranched alkyl radical having 8 to 18 carbon atoms and $Z_n$ is an oligoglycosyl radical consisting of 1 to 2 hexose or pentose units or mixtures thereof,
b) as carboxymethylated fatty alcohol oxyethylates, compounds of the formula (II)

$$R'\text{-}(OC_2H_4)_mO\text{-}CH_2\text{-}COOM \qquad (II)$$

are employed, in which R' is a linear or branched, saturated aliphatic radical having 10 to 18 carbon atoms, m is from 1 to 11 and M is an alkali metal, alkaline earth metal, hydrogen, ammonium or alkylammonium ion, the degree of carboxymethylation being from 60 to 100% by weight,

c) as monoalkyl sulphosuccinates, compounds of the formula (III)

$$\begin{array}{l} R''\text{-O-CO-CH}_{(2)}\text{-CH}_{(2)}\text{-COOMe} \\ \qquad\qquad\qquad\qquad | \\ \qquad\qquad\qquad\quad SO_3Me \end{array} \qquad (III),$$

are employed, in which R" is a saturated or unsaturated, branched or unbranched alkyl radical having 8 to 18 carbon atoms or an alkyloxyethyl radical of the formula IV

$$R'''(OCH_2CH_2)_p\text{-} \qquad (IV),$$

in which R''' is a saturated, branched or unbranched alkyl radical having 10 to 18 carbon atoms, p is from 1 to 8 and Me is an alkali metal, alkaline earth metal, hydrogen, ammonium or alkylammonium ion,
and d) as surfactant additives, organic sulphates or ether sulphates, organic sulphonates, sarcosinates, taurides, betaines, protein hydrolysates, quaternary surfactants or di- and oligo fatty acid eaters of oxyethylated polyhydric alcohols and all mixtures thereof are added.

**2.** A surfactant combination according to claim 1, characterized in that, as alkyl polyglycoside, an alkyl glucoside having a saturated, unbranched alkyl radical of 10 to 16 carbon atoms and a degree of glucosidation of from 1.1 to 1.6 is used.

**3.** A surfactant combination according to claim 1, characterized in that R' is a linear, saturated aliphatic radical having 12 to 16 carbon atoms, m is from 2 to 8 and M is an alkali metal or ammonium ion, the degree of carboxymethylation being from 70 to 100%.

**4.** A surfactant combination according to claim 1, characterized in that R" is an alkyl oxyethylate having 12 to 18 C atoms in the linear, saturated alkyl chain, the degree of oxyethylation is from 2 to 6 and Me is an alkali metal or ammonium ion.

**5.** A surfactant combination according to any one of claims 1 to 4, characterized in that, as electrolytic thickening agents, sodium chloride, ammonium chloride, sodium sulphate or magnesium sulphate are employed in an amount from 0.5 to 5%.

## Revendications

**1.** Combinaisons de tensioactifs épaississables par un électrolyte, pour gels bains, shampooings et nettoyants cosmétiques, contenant

a) de 7 à 25 % en poids d'un alkylpolyglycoside,
b) de 3 à 25 % en poids d'un produit d'éthoxylation carboxyméthylé d'un alcanol,
c) de 0 à 25 % en poids d'un monoalkylsulfosuccinate,
d) de 0 à 3 % d'additifs tensioactifs,

caractérisées en ce que :
le rapport des quantités a:b ou a:(b+c) est compris entre 4:6 et 7:3, le rapport (a+b):d ou (a+b+c):d n'est pas inférieur à 5, et le rapport b:c est compris entre 1:8 et 10:0,

a) on utilise comme alkylpolyglycosides des composés de formule (I)

$$R\text{-O-}Z_n \qquad (I)$$

dans laquelle R est un radical alkyle à chaîne droite ou ramifiée, saturé ou insaturé, ayant de 8 à 18 atomes de carbone, $Z_n$ est un radical oligoglycosyle constitué de 1 à 2 motifs hexose ou pentose, ou leurs mélanges,

b) on utilise comme produits d'éthoxylation carboxyméthylés d'alcools gras des composés de formule (II)

$$R'\text{-}(OC_2H_4)_mO\text{-}CH_2\text{-COOM} \qquad (II)$$

dans laquelle R' est un radical aliphatique saturé à chaîne droite ou ramifiée ayant de 10 à 18 atomes de carbone, m vaut de 1 à 11, M est un ion métal alcalin, métal alcalino-terreux, hydrogène, ammonium ou alkylammonium, le degré de carboxyméthylation étant compris entre 60 et 100 % en poids,

c) on utilise comme monoalkylsulfosuccinates des composés de formule (III)

$$R''-O-CO-CH_{(2)}-\underset{SO_3Me}{\underset{|}{CH_{(2)}}}-COOMe \qquad (III)$$

dans laquelle R" est un radical alkyle à chaîne droite ou ramifiée, saturé ou insaturé, ayant de 8 à 18 atomes de carbone, ou un radical alkyléthoxy de formule (IV)

$$R'''(OCH_2CH_2)_p- \qquad (IV)$$

dans laquelle R'" est un radical alkyle saturé à chaîne droite ou ramifiée ayant de 10 à 18 atomes de carbone, et p vaut de 1 à 8, Me est un ion métal alcalin, métal alcalino-terreux, hydrogène, ammonium ou alkylammonium,

et d) en tant qu'additifs tensioactifs, on ajoute des sulfates et éthersulfates organiques, des sulfonates organiques, des sarcosinates, des taurides, des bétaïnes, des produits d'hydrolyse de protéines, des tensioactifs quaternaires ainsi que des esters de diacides et d'oligoacides gras de polyalcools éthoxylés.

**2.** Combinaison de tensioactifs selon la revendication 1, caractérisée en ce que l'on utilise comme alkylpolyglycoside un alkylpolyglucoside ayant un radical alkyle saturé à chaîne ramifiée ayant de 10 à 16 atomes de carbone et un degré de glucosidation compris entre 1,1 et 1,6.

**3.** Combinaison de tensioactifs selon la revendication 1, caractérisée en ce que R' est un radical aliphatique saturé linéaire ayant de 12 à 16 atomes de carbone, m vaut de 2 à 8 et M est un métal alcalin ou l'ammonium, le degré de carboxyméthylation étant de 70 à 100 %.

**4.** Combinaison de tensioactifs selon la revendication 1, caractérisée en ce que R" est un produit d'éthoxylation d'un alcanol ayant de 12 à 18 atomes de carbone dans la chaîne alkyle saturée linéaire, le degré d'éthoxylation est de 2 à 6 et Me désigne un métal alcalin ou l'ammonium.

**5.** Combinaison de tensioactifs selon l'une des revendications 1 à 4, caractérisée en ce qu'on utilise comme épaississant électrolytique du chlorure de sodium, du chlorure d'ammonium, du sulfate de sodium ou du sulfate de magnésium en des quantités de 0,5 à 5 % en poids.

Abb.1: Viskosität, Reibschaum, Zeinzahl von $C_{12}C_{16}$-$APG_{1,3}$/$C_{12}C_{14}(EO)_4CH_2COONa$ Mischungen (20% WAS, 1,5% NaCl)

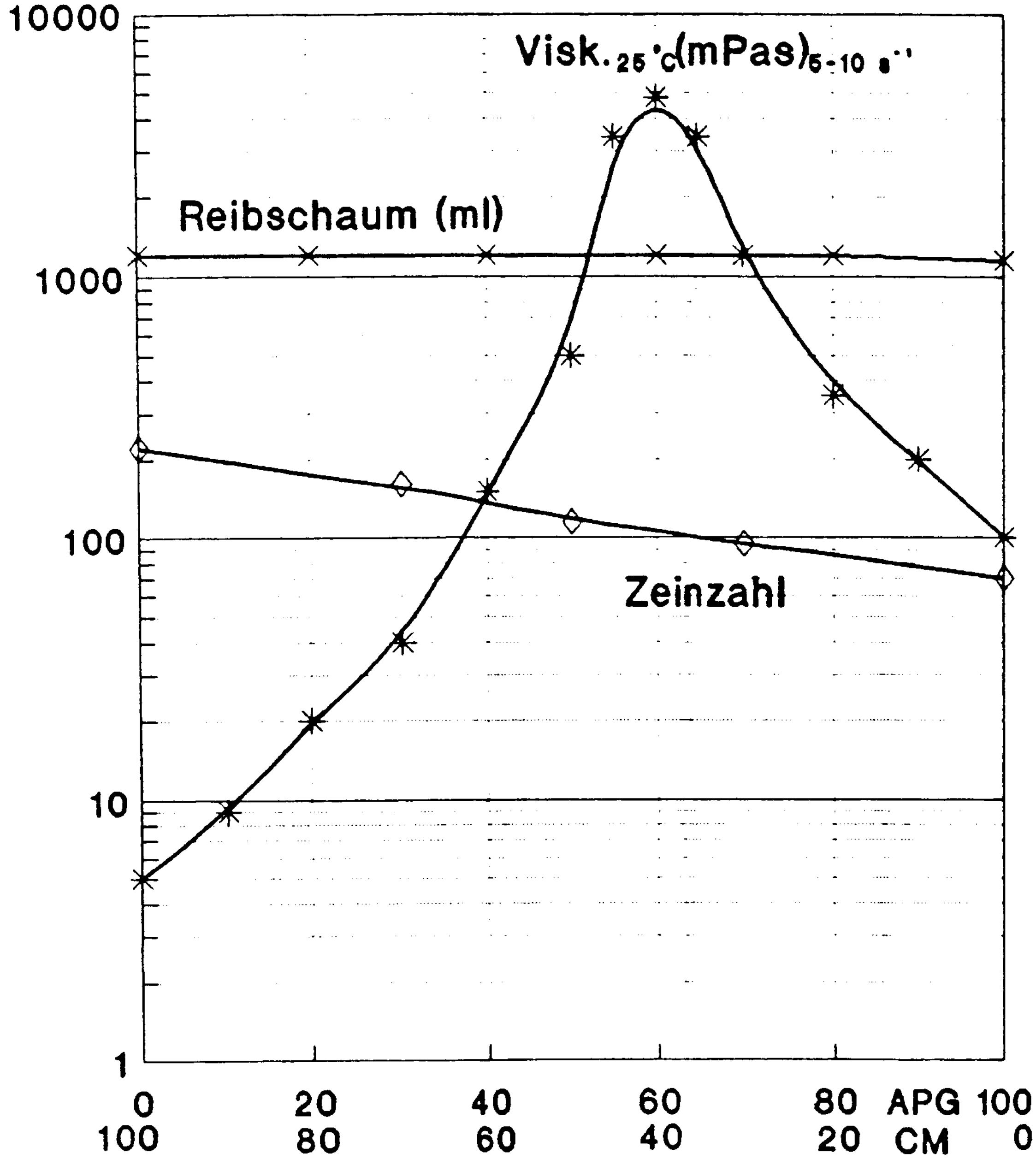

Abb. 2: Verdickbarkeit durch NaCl
(waschaktive Substanz 20 %)

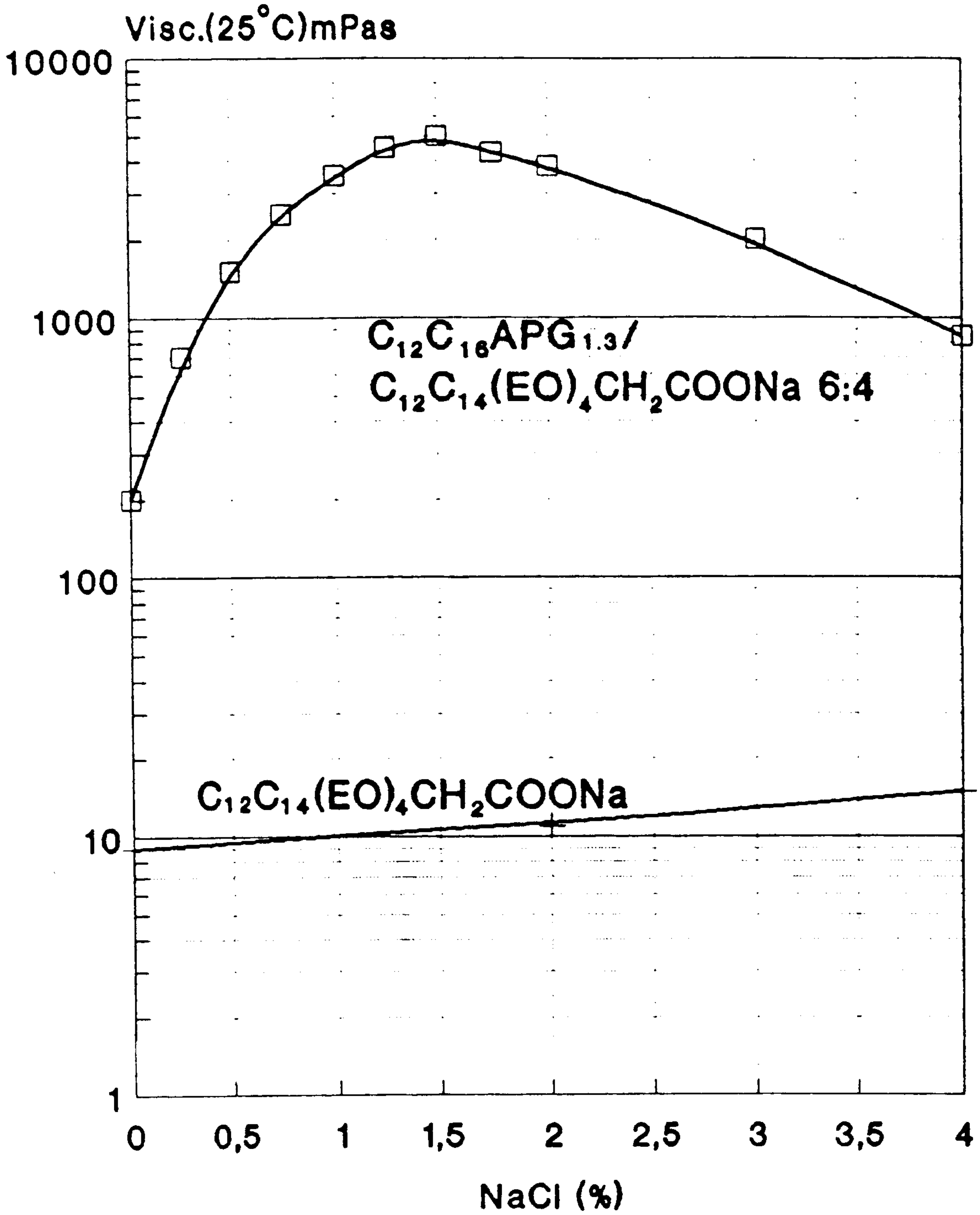

Abb.3: Viskosität in Abhängigkeit der Zusammensetzung

$C_{12}C_{14}$-$APG_{1.3}$/$C_{12}C_{14}(EO)_2CM$/$C_{12}C_{14}(EO)_4CM$

20% WAS, 2% NaCl, 25°C

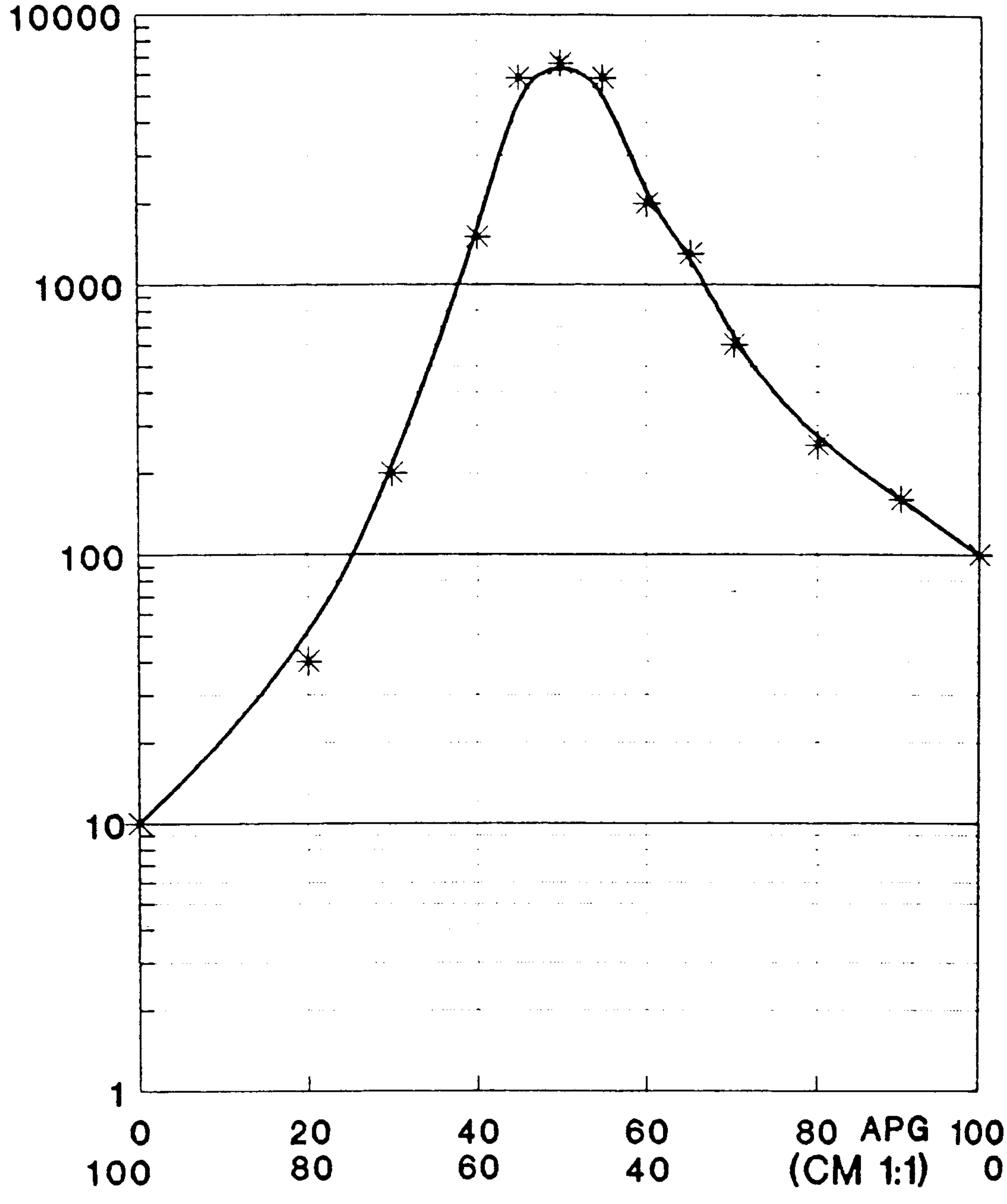

Abb.4: Viskosität in Abhängigkeit der Zusammensetzung

$C_{12}C_{14}$-$APG_{1,3}$/$C_{12}C_{14}(EO)_4CM$/$C_{12}C_{14}(EO)_3SS$

15% WAS, 2% NaCl, 25°C

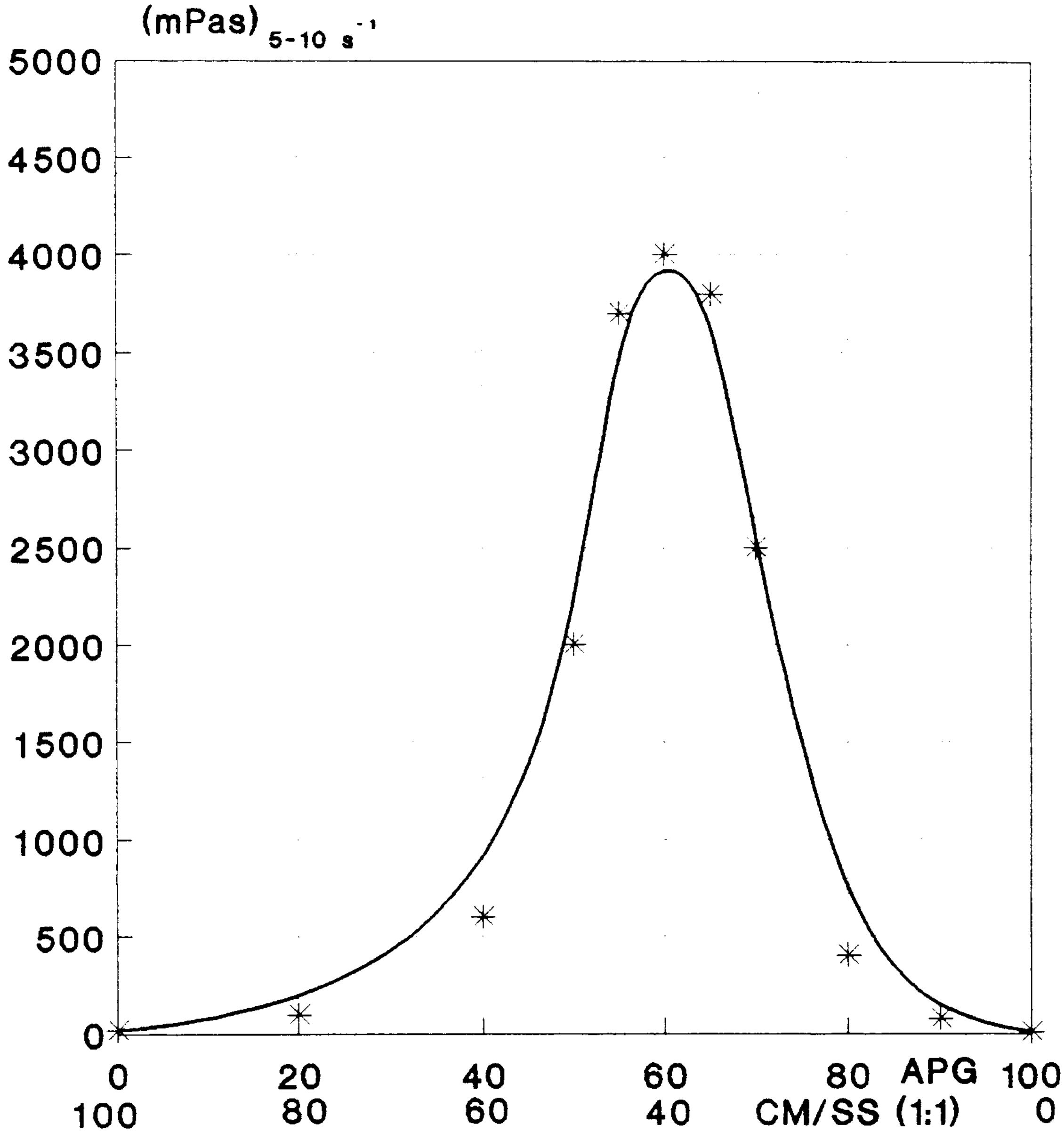

Abb. 5: Verdickbarkeit durch Elektrolyt
(waschaktive Substanz 15%, 25 °C)